# EUROPÄISCHE PATENTSCHRIFT

(11) **EP 0 631 774 B1**
(45) Veröffentlichungstag und Bekanntmachung des Hinweises auf die Patenterteilung: **08.01.1997**
(21) Anmeldenummer: 94109481.5
(22) Anmeldetag: 20.06.1994
(51) Int. Cl.: A61K 9/113, A61K 7/00, B01F 17/00

(54) **Flüssige oder pastöse, lagerstabile, multiple Emulsion des Typs W1/O/W2**
Fluid or pasty, storage-stable, W1/0/W2 type multiple emulsion
Emulsion multiple de type W1/0/W2, stable au stockage, fluide ou pâteuse

(30) Priorität: 03.07.1993 DE 4322174
(43) Veröffentlichungstag der Anmeldung: 04.01.1995
(73) Patentinhaber: Th. Goldschmidt AG, D-45127 Essen (DE)
(72) Erfinder: Grüning, Burghard, Dr., D-45134 Essen (DE); Hameyer, Peter, D-45138 Essen (DE); Weitemeyer, Christian, Dr., D-45134 Essen (DE)

(56) Entgegenhaltungen:
- EP-A- 0 174 377
- EP-A- 0 176 884
- EP-A- 0 278 103
- EP-A- 0 345 075
- EP-A- 0 386 507
- EP-A- 0 540 857
- EP-A- 0 559 013
- WO-A-93/02666
- FR-A- 2 326 914
- FR-A- 2 645 740

## Beschreibung

Die Erfindung betrifft eine flüssige oder pastöse, lagerstabile, multiple Emulsion des Typs W¹/O/W², wobei W² die äußere Phase der multiplen Emulsion bildet, an der Grenzfläche W¹/O ein hydrophober Emulgator und an der Grenzfläche O/W² ein hydrophiler Emulgator vorhanden ist.

Die Erfindung betrifft insbesondere langzeitstabile, hochviskose, multiple Emulsionen des W¹/O/W²-Typs, die sich für kosmetische und pharmazeutische Anwendungen eignen und deren Gehalt an lipidartigen Substanzen, d.h. Ölen und Fetten, max. 35 % beträgt.

Multiple W¹/O/W²-Emulsionen unterscheiden sich von zweiphasigen O/W-Emulsionen dadurch, daß in der äußeren Wasserphase W² Öltröpfchen dispergiert sind, die ihrerseits feinstteilige Wassertröpfchen (W¹) enthalten. Die Zusammensetzung der äußeren Wasserphase W² und der inneren Wasserphase W¹ kann gleich, aber auch unterschiedlich sein. Den O/W- und W/O/W-Emulsionen ist gemeinsam, daß sie, im Gegensatz zur W/O-Form, in Wasser verteilbar sind.

An multiplen W¹/O/W²-Emulsionen besteht in der kosmetischen und pharmazeutischen Industrie ein prinzipielles Interesse, weil sie neue Möglichkeiten zur Formulierung von hautpflegenden oder pharmazeutischen Präparaten eröffnen. So lassen sich sowohl öl- als auch wasserlösliche empfindliche Wirkstoffe in den inneren Emulsionsphasen plazieren. Dadurch vermindert sich die Gefahr von oxidativen Veränderungen oder Wechselwirkungen mit dem Verpackungsmaterial.

Weiterhin bieten sie die grundsätzliche Voraussetzung dafür, zwei wasserlösliche, jedoch miteinander unverträgliche Substanzen in einem Präparat einbringen zu können.

Ein anderer interessanter Aspekt sind hochviskose, in Wasser verteilbare Emulsionen, welche über die für hautpflegende und pharmazeutische Präparate bevorzugte dickflüssige bis cremeartige Konsistenz verfügen, deren äußere Wasserphase jedoch nicht mittels sog. Konsistenzgeber entscheidend verdickt werden muß. Entsprechende zweiphasige O/W-Emulsionen enthalten aus diesem Grund Hilfsmittel, welche die Viskosität der Wasserphase bis auf cremeartige bzw. dickflüssige Konsistenz anheben. Üblich sind Mischungen von polaren Wachsen (z.B. Glycerinstearate, gesättigte Fettalkohole) und hydrophilen Emulgatoren (z.B. Alkalistearate, Polyethylenglykolstearylether) und/oder wasserlösliche Organopolymere (Polyacrylate, Cellulosederivate).

Aufgrund der erforderlichen, relativ hohen Dosierung beeinträchtigen diese viskositätserhöhenden Zusätze das Applikationsverhalten zahlreicher Formulierungen, weil sie ein klebriges bzw. wachsartig/stumpfes Hautgefühl erzeugen. O/W-Emulsionen, deren äußere Wasserphase keine oder nur geringe Mengen viskositätserhöhender Substanzen enthält, sind bekanntlich nur dann von hochviskoser Beschaffenheit, wenn der Gehalt an disperser Ölphase mehr als 70 Vol.-% beträgt. In diesem Fall ist der Abstand zwischen den dispergierten Öltröpfchen so gering, daß durch Wechselwirkungskräfte ihre Mobilität stark verringert oder faktisch aufgehoben wird. An Formulierungen, die mehr als 70 Vol.-% Ölphase enthalten, besteht jedoch wegen ihren sehr öligen Charakters wenig Interesse, insbesondere betrifft das kosmetische Anwendungen.

Bei der multiplen W¹/O/W²-Form kann die angestrebte hochviskose Konsistenz erreicht werden, ohne die äußere Wasserphase entscheidend zu verdicken, wenn es gelingt, entsprechend große Anteile der W/O-Primäremulsion einzubringen. Die Zusammensetzung der letzteren muß jedoch so beschaffen sein, daß der Ölphasengehalt im Endprodukt nicht oberhalb des insbesondere für Hautpflegepräparate bevorzugten Bereichs von 15 bis 35 Gew.-% liegt.

Die Herstellung stabiler W¹/O/W²-Emulsionen ist ungleich schwieriger als die von zweiphasigen Emulsionen. Emulgatoren, emulsionsstabilisierende Hilfsmittel und Emulgiermethode müssen daher sehr sorgfältig aufeinander abgestimmt sein. Andernfalls haben die Emulsionen ungenügende Stabilität oder die multiple Form kommt überhaupt nicht zustande. Es bilden sich entweder zweiphasige W¹,W²/O-Emulsionen, d.h. W² geht vollständig in die disperse Wasserphase W¹ über, oder ein erheblicher Teil der W¹/O-Emulsionströpfchen zersetzt sich unter Bildung einer instabilen, dünnflüssigen O/W¹,W²-Emulsion. Die zweiphasige W/O-Form ist daran zu erkennen, daß sie in Wasser nicht verteilbar ist. Instabile W¹/O/W²-Emulsionen gehen allmählich in eine der beschriebenen Formen über.

Die Methoden, die in der Fach- und Patentliteratur zur Herstellung von multiplen W¹/O/W²-Emulsionen erwähnt werden oder die aus theoretischen Überlegungen in Frage kommen, sind überwiegend zweistufig. Als besonders zuverlässig hat sich die folgende Methode erwiesen:

Als Primäremulsion dient eine sehr feinteilige, stark belastbare W/O-Emulsion, die durch die Einarbeitung der Wasserphase W¹ in die emulgatorhaltige Ölphase O gewonnen wird, ggf. unter Wärmezufuhr. Diese wird im zweiten Verfahrensschritt in die wäßrige Phase W² eingebracht, in der ein die Dispergierung begünstigender hydrophiler Emulgator und/oder ein emulsionsstabilisierendes Organopolymer enthalten sind.

Die Patentschrift DE-PS 33 90 056 betrifft W/O/W-Emulsionen, für deren Stabilität es günstig ist, wenn die W/O-Primäremulsion durch Inversion einer O/W-Emulsion hergestellt wird. Die Funktion des hydrophilen und des lipophilen Emulgators wird von Polyglycerin-Polyrizinoleaten ausgeübt. Diese Emulsionen sind jedoch bei erhöhten Lagertemperaturen, z.B. 40°C, nicht stabil.

Gemäß der Offenlegungsschrift DE-OS 41 36 699 bilden sich stabile W/O/W-Emulsionen, wenn in die W/O-Primäremulsion so viel einer wäßrigen Tensidlösung eingerührt ist, bis eine Inversion erfolgt. Hierbei dürfte es sich um eine partielle Inversion handeln, durch die ein Teil der dispersen Wasserphase freigesetzt wird, unter Bildung einer kontinuierlichen Wasserphase, in der die W/O-Partikel eingebettet sind. Als Stabilisierungsmittel, das die Viskosität der Wasserphase erhöht, werden erhebliche Zusatzmengen eines Verdickungsmittels benötigt. An Emulgatoren und Stabilisatoren werden Methoxypolyethylenglykol-Dodecylglykol-Copolymere, Polyethylenglykol-Dodecylglykol-Copolymere sowie Ethylenoxid-Propylenoxid-Blockpolymere und als Gelierungsmittel Polyglycerylmethacrylate genannt. Die hohe Dosierung des in der äußeren Wasserphase eingesetzten Verdickungsmittels führt jedoch zu einer Beeinträchtigung der Applikationseigenschaften.

Ein einstufiges Herstellungsverfahren für multiple W/O/W-Emulsionen kann der DE-OS 41 31 678 entnommen werden. Dort werden als hydrophile und lipophile Emulgatoren Polyethylenglykolderivate von Fettsäuren und Fettalkoholen genannt. Aus den Rezepturbeispielen geht hervor, daß die Emulgatordosierung relativ hoch sein muß, d.h. 4 bis 8 %, bezogen auf multiple Emulsion.

Die Erfindung befaßt sich mit dem technischen Problem, stabile, dickflüssige bis cremeartige W¹/O/W²-Emulsionen zu entwickeln, die max. 35 % Ölphase enthalten und für deren Bildung nur geringe Zusatzmengen von Emulgatoren und insbesondere wasserlöslichen, emulsionsstabilisierenden Hilfsmitteln erforderlich sind.

Es wurde gefunden, daß Emulsionen des Typs W¹/O/W², wobei W² die äußere Phase der multiplen Emulsion bildet, an der Grenzfläche W¹/O ein hydrophober Emulgator und an der Grenzfläche O/W² ein hydrophiler Emulgator vorhanden ist, diese Eigenschaften aufweisen, wenn erfindungsgemäß
(1) der hydrophobe Emulgator einen HLB-Wert ≤ 8 aufweist und
   a₁) ein Polyacrylsäureester mit langkettigen Kohlenwasserstoff- und Polyoxyalkylengruppen oder
   a₂) ein Polyoxyalkylenpolysiloxan mit langkettigen Alkylresten ist, und der hydrophobe Emulgator in einer Menge von 0,3 bis 1,5 Gew.%, bezogen auf multiple Emulsion, enthalten ist, und
(2) der hydrophile Emulgator einen HLB-Wert > 8 aufweist und
(3) die Ölphase O in einer Menge von ≤ 35 Gew.-%, bezogen auf Gesamtemulsion, enthalten ist.

Das von William C. Griffin formulierte HLB-System (J. Soc. Cosm. Chemists 5, S. 249, 1954), das sich zunächst nur auf Fettsäurepolyolpartialester und Polyethylenglykolderivate bezog, ermöglicht eine Klassifizierung der Emulgatoren nach ihrem Verhalten in Wasser:

| HLB-Wert | Verhalten bei Zugabe von Wasser |
|---|---|
| 1 bis 4 | nicht wasserdispergierbar |
| 3 bis 6 | sehr schlechte Dispergierung |
| 6 bis 8 | milchige Dispersion nach kräftigem Schütteln |
| 8 bis 10 | stabile, milchige Dispersion |
| 10 bis 13 | durchscheinende, klare Dispersion |
| über 13 | klare, kolloidale Lösung |

Da sich der HLB-Wert (Lipophil-Hydrophil-Balance) von polymeren Emulgatoren nach der Griffin-Formel nicht berechnen läßt, wurde dieser Wert aus dem Verhalten gegenüber Wasser abgeleitet.

Der hydrophobe Emulgator ist zunächst durch seinen HLB-Wert gekennzeichnet. Der HLB-Wert soll vorzugsweise ≤ 8, bevorzugt ≤ 6 sein. Ein weiteres Merkmal des hydrophoben Emulgators besteht darin, daß es sich bei ihm um eine polymere Verbindung handelt, die aus zwei verschiedenen Verbindungsklassen ausgewählt ist:

Der hydrophobe Emulgator kann ein Polyacrylsäureester mit langkettigen Kohlenwasserstoff- und Polyoxyalkylengruppen sein.

Ein Emulgator dieses Typs und dieser Struktur ist in der DE-OS 39 06 702 beschrieben. Ein solcher Polyacrylsäureester ist erhältlich durch Umesterung von durch radikalische Polymerisation erhaltenen Polyacrylsäurealkylestern, deren Alkylgruppen 1 bis 4 Kohlenstoffatome aufweisen, mit einem Gemisch von
a) Alkoholen, ausgewählt aus der Gruppe bestehend aus
   a1 gesättigten aliphatischen Alkoholen mit 4 bis 22 Kohlenstoffatomen,
   a2 ungesättigten aliphatischen Alkoholen mit 8 bis 22 Kohlenstoffatomen,
   a3 Alkylphenolen, deren Alkylgruppen jeweils 8 bis 12 Kohlenstoffatome aufweisen oder deren Oxalkylierungsprodukte mit 1 bis 3 Oxyethylen- und/oder Oxypropyleneinheiten und
b) Polyoxyalkylenmonoolen der allgemeinen, durchschnittlichen Formel

   R¹O-(CₙH₂ₙO-)ₓH

   wobei
   - R¹: der Kohlenwasserstoffrest eines Startalkohols R¹OH ist,
   - n: 2, 3 oder 4 ist und in der durchschnittlichen Polyoxyalkylengruppe einen mittleren Wert von 2 bis 3 hat,
   - x: in der durchschnittlichen Polyoxyalkylengruppe einen mittleren Wert von 4 bis 50 hat,
wobei das molare Verhältnis von a : b und der Wert der Indices n und x so gewählt ist, daß das Verfahrensprodukt einen HLB-Wert von ≤ 8 aufweist,
in solchen Mengen, daß bis zu 70 % der Estergruppen umgesetzt werden, in Gegenwart eines an sich bekannten Umesterungskatalysators bei Temperaturen von 70 bis 160°C, gegebenenfalls in Gegenwart eines Lösungsmittels.

Es ist dabei dem Fachmann klar, daß der HLB-Wert des Polyacrylsäureesters auch von der Kettenlänge des Alkoholes a) abhängt.

Der hydrophobe Emulgator kann andererseits ein Polyoxyalkylenpolysiloxan mit langkettigen Alkylresten sein. Hierbei handelt es sich in erster Linie um lineare Organopolysiloxane, welche seiten- und/oder endständig sowohl langkettige Alkylreste wie Polyetherreste aufweisen. Der HLB-Wert dieser Verbindungen ergibt sich aus der Hydrophilie des Polyetherrestes, der Kettenlänge des Siloxans und der Kettenlänge des langkettigen Alkylrestes. Beispiele geeigneter modifizierter Polysiloxane sind in der EP-PS 0 125 779 und der EP-PS 0 176 884 beschrieben. Die modifizierten Polysiloxane können dabei der folgenden Formel entsprechen: 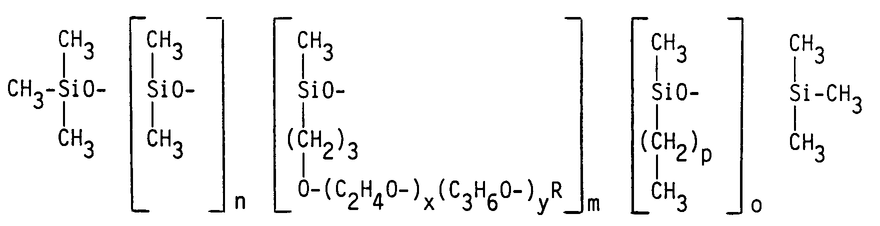 wobei
R ein niederer Alkylrest oder ein Wasserstoffrest ist,
n = 10 bis 200,
m = 1 bis 25,
o = 1 bis 100,
p = 7 bis 17 und
x und y ganze Zahlen von 1 bis 200 sind.

Die Kohlenstoffkette des langkettigen Alkylrestes kann durch einen Ethersauerstoff unterbrochen sein. Derartige Verbindungen sind in der offengelegten europäischen Patentanmeldung 0 459 705 beschrieben.

Die Dosierung der hydrophoben Emulgatoren beträgt 0,3 bis 1,5 Gew.-%, bezogen auf multiple Emulsion. Aufgrund ihres lipophil/hydrophoben Charakters üben die hydrophoben Emulgatoren keinerlei Einfluß auf die Viskosität der äußeren Wasserphase W² aus.

Vorzugsweise enthält die äußere Wasserphase W² hydrophile Emulgatoren eines HLB-Wertes ≥ 13 in einer Konzentration von ≥ 0,05 Gew.-% sowie emulsionsstabilisierende Hilfsmittel in einer Konzentration von ≥ 0,05 Gew.-%.

Dabei sind als hydrophile Emulgatoren solche aus der Gruppe der Amphotenside, Sulfosuccinate, Fettsäuresarcoside, Fettsäure-Eiweiß-Kondensate, Isothionate, Taurate und der aus Zuckern abgeleiteten Tenside bevorzugt. Ihre überraschend gute Wirkung dürfte darauf beruhen, daß sie wegen ihres ausgeprägten hydrophilen Charakters nicht in die Ölphase O migrieren können und daß sie schon in einer geringen Dosierung befähigt sind, die Dispergierung der W/O-Primäremulsion ausreichend zu unterstützen, ohne andererseits die W/O-Partikel zu zerstören.

Vorzugsweise enthält die äußere Wasserphase W² als emulsionsstabilisierende Hilfsmittel Organopolymere aus der Gruppe der Cellulosederivate, der Carboxyl-/Carboxylatgruppen enthaltenden Polyacrylate, u.a. solche, die in der DE-PS 39 25 220 beschrieben sind, oder Xanthangum. Diese können der Wasserphase W² vor oder nach der Emulsionsbildung zugesetzt werden.

Die innere Wasserphase W¹ enthält vorteilhaft emulsionsstabilisierende Hilfsmittel, insbesondere Elektrolyte, z.B. in Form von Natriumchlorid, dessen Konzentration zwischen 0,2 und 3,0 Gew.-% liegen kann.

Sowohl die innere Wasserphase W¹ wie auch die äußere Wasserphase W² können vorzugsweise Substanzen mit kosmetischer oder pharmazeutischer Wirkung, wie Harnstoff, Natriumlactat, Natriumpyrrolidoncarboxylat, Dihydroxyaceton oder Panthenol, enthalten.

Die Ölphase O enthält vorzugsweise neben den hydrophoben Emulgatoren Öle und Fette mit hautpflegenden Eigenschaften, wie Kohlenwasserstoffe, z.B. Paraffinöle und Vaselinen, Fettsäureester, wie Pflanzenöle, Isopropylmyristat und Decyloleat, Ester, die aus linearen oder verzweigten Säuren und Alkohlen gewonnen werden, wie Ethylhexylethylhexanat, Diethylhexyladipat und Isopropylisostearat und Siliconprodukte, wie Polydimethylsiloxane, Cyclodimethylsiloxane und Polymethylalkylsiloxane oder Mischungen derselben, oder besteht aus diesen Produkten. Weiterhin können emulsionsstabilisierende Additive enthalten sein, insbesondere Wachse mit Schmelzpunkten > 60°C oder Metallseifen sowie Wirkstoffe mit kosmetischer oder pharmazeutischer Wirkung, z.B. Tocopherolacetat, Nicotinsäureester und UV-Filter.

Die multiplen Emulsionen sind in Wasser verteilbar, und sie weisen die angestrebte dickflüssige bzw. cremeartige Konsistenz auf. Diese beruht auf dem hohen Gehalt an dispergierter W/O-Primäremulsion, denn die in der Wasserphase W² eingesetzten Organopolymere bewirken mit ca. 1000 bis 3000 mPas nur eine vergleichsweise geringe Viskositätserhöhung. Ein wesentlicher Vorteil der erfindungsgemäßen lipophilen Emulgatoren besteht darin, daß geringe Dosierungen, wie sie in der Praxis erwünscht sind, ausreichen.

Die Herstellung der erfindungsgemäßen multiplen W¹/O/W²-Emulsionen kann auf folgende Weise geschehen:

### 1. W¹/O-Primäremulsion

Das Gewichtsverhältnis zwischen der Ölphase O und der Wasserphase W¹ wird vorzugsweise so eingestellt, daß sich eine hochviskose, jedoch fließfähige Emulsion bilden kann. Hierzu wird auf "Tenside", Surfactants, Detergents, 29. Jahrgang, 1992/2, S. 78 bis 83, verwiesen.

Emulsionen mit cremeartiger Konsistenz sind ebenfalls verwendbar. Es ist jedoch schwieriger, diese in Wasser zu dispergieren, d.h. in die multiple Form zu überführen. Die Emulgierung kann in der Weise erfolgen, daß die auf 80°C erhitzte Ölphase O vorgelegt und die Wasserphase W¹, deren Temperatur ca. 20°C beträgt, unter intensivem Rühren eingearbeitet wird. Erforderlich sind Rührer mit starker Scherkraftentwicklung. Wasserlösliche Wirkstoffe, die in der inneren Wasserphase eingeschlossen werden sollen, werden zuvor in der Wasserphase W¹ gelöst.

### 2. Überführung in die multiple Form

Sie erfolgt bei einer Temperatur von 5 bis 90°C, bevorzugt von 15 bis 30°C, durch allmähliches Einrühren der W¹/O-Primäremulsion in eine wäßrige Lösung, die einen hydrophilen Emulgator und/oder ein emulsionsstabilisierendes Organopolymer enthält. In dieser Verfahrensstufe genügt mäßiges Rühren. Eine intensive mechanische Bearbeitung mittels Emulgiermaschine könnte den Übergang der W¹/O/W²-Form in die zweiphasige W¹,W²/O-Form zur Folge haben. Die wasserlöslichen Substanzen mit pharmazeutischer oder kosmetischer Wirkung, die in der externen Phase enthalten sein sollen, werden nach Bildung der multiplen W¹/O/W²-Form eingerührt.

### 3. Rezepturbeispiele

Die W/O-Primäremulsion besteht aus den unter A (Ölphase O) und B (Wasserphase W¹) aufgeführten Komponenten. C gibt die Zusammensetzung der wäßrigen Lösung (W²) an, in welcher die Primäremulsion dispergiert wird. Die in den Rezepturen angegebenen Zahlenwerte entsprechen dem prozentualen Anteil der Substanzen.

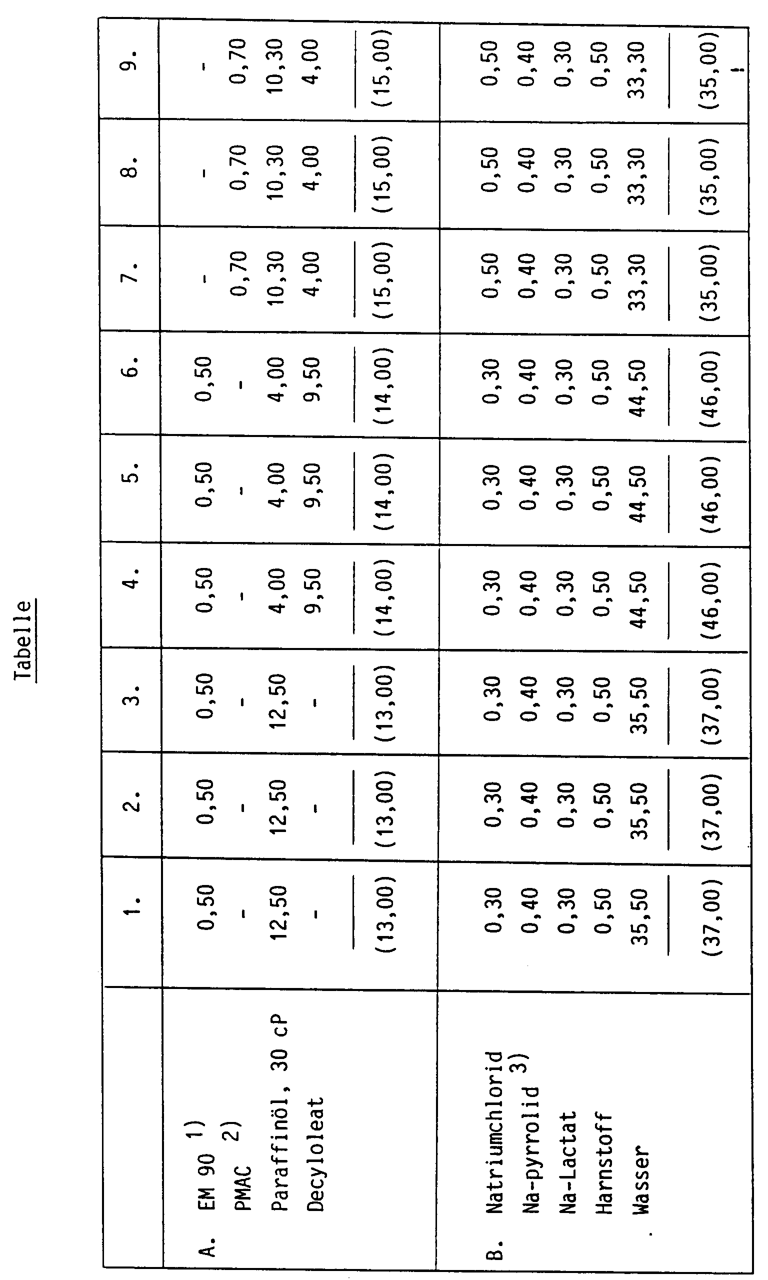

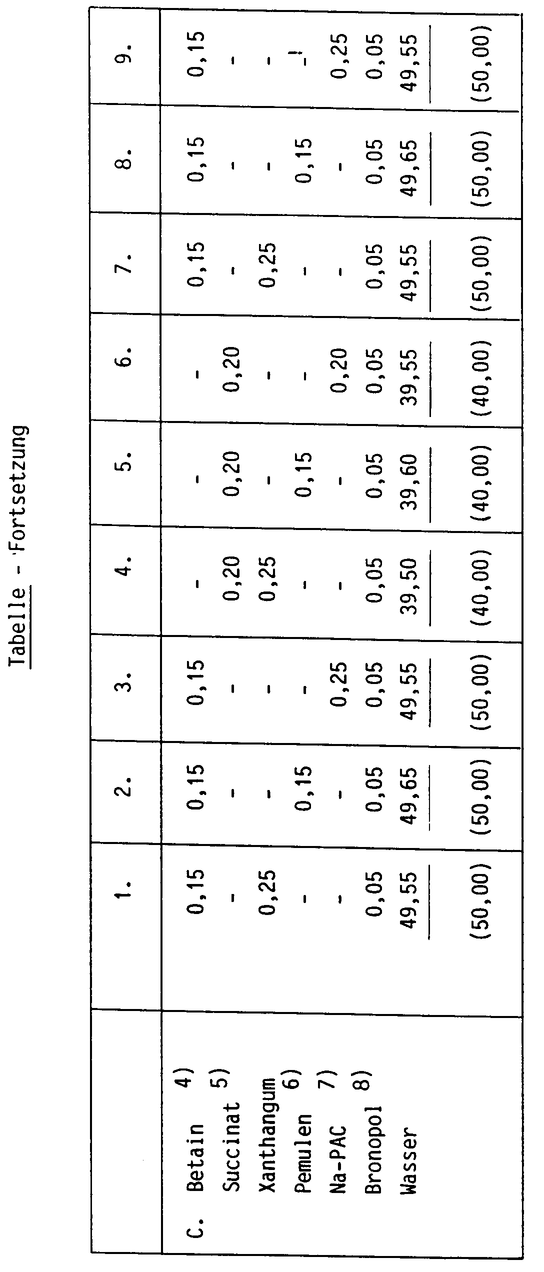

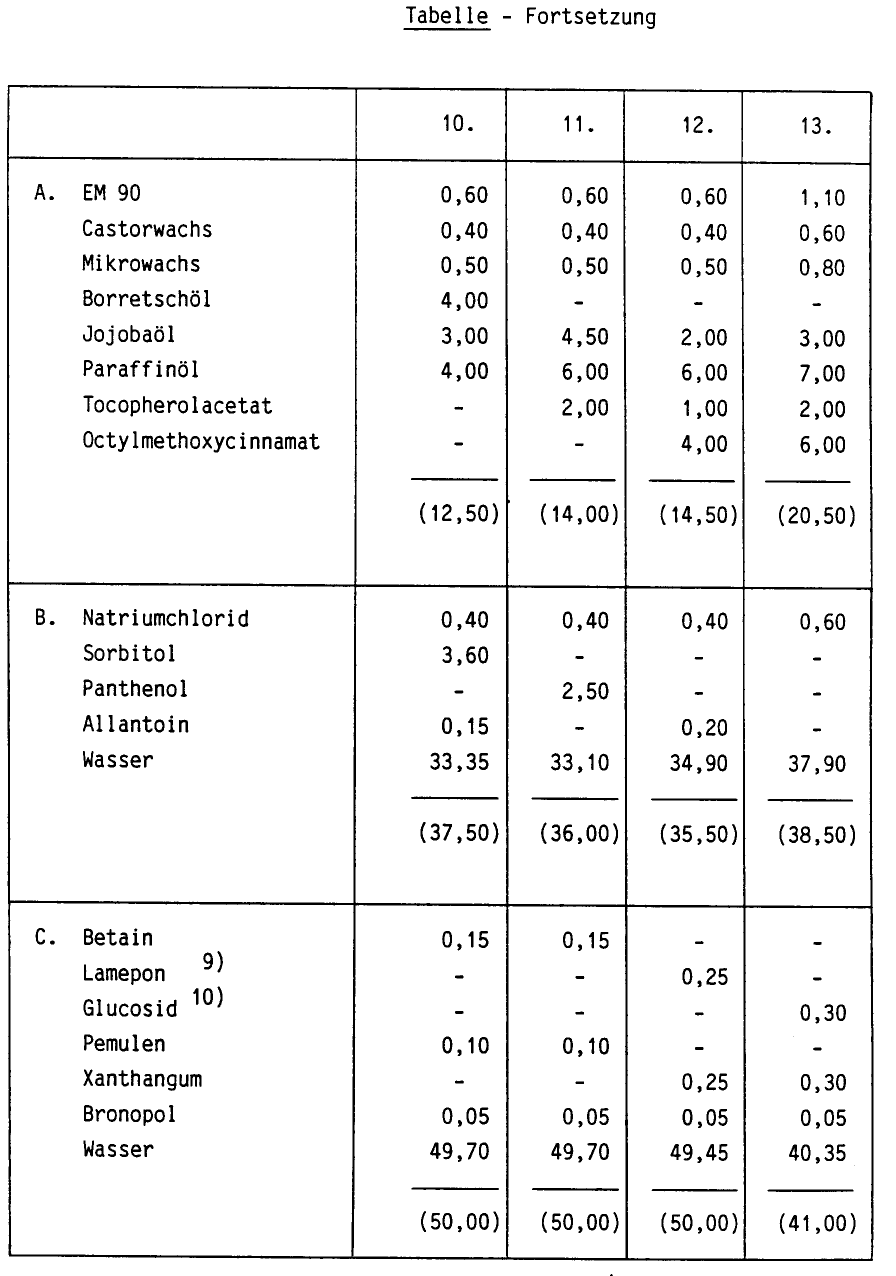

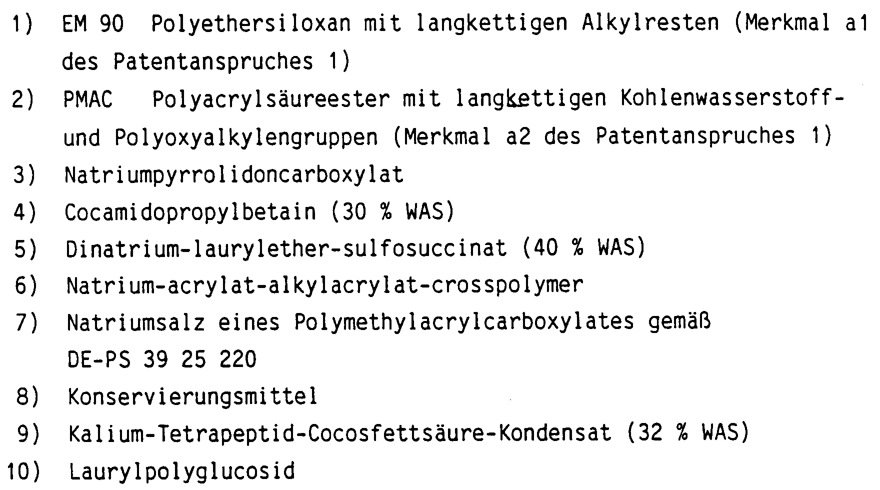

Die Emulsionspräparate Nr. 1-13 sind in Wasser verdünnbar. Sie haben stark dickflüssige bzw. cremeartig-weiche Konsistenz. Nach dreimonatiger Lagerung bei 20 °C bzw. vierwöchiger Lagerung bei 40 °C waren weder Zersetzungserscheinungen noch der Übergang in die binäre, in Wasser nicht verdünnbare W/O-Form festzustellen. Die W/O/W-Form ist an der Verdünnbarkeit in Wasser und an der dickflüssigen bis cremeartigen Konsistenz zu erkennen. Die als äußere Phase fungierenden wäßrigen Lösungen (C) sind dagegen mit einer durchschnittlichen Viskosität von 400 mPa.s niedrigviskos.

## Patentansprüche

1. Flüssige oder pastöse, lagerstabile, multiple Emulsion des Typs W¹/O/W², wobei W² die äußere Phase der multiplen Emulsion bildet, an der Grenzfläche W¹/O ein hydrophober Emulgator und an der Grenzfläche O/W² ein hydrophiler Emulgator vorhanden ist, dadurch gekennzeichnet, daß
(1) der hydrophobe Emulgator einen HLB-Wert ≤ 8 aufweist und
a₁) ein Polyacrylsäureester mit langkettigen Kohlenwasserstoff- und Polyoxyalkylengruppen oder
a₂) ein Polyoxyalkylenpolysiloxan mit langkettigen Alkylresten ist, und der hydrophobe Emulgator in einer Menge von 0,3 bis 1,5 Gew.-%, bezogen auf multiple Emulsion, enthalten ist, und
(2) der hydrophile Emulgator einen HLB-Wert > 8 aufweist und
(3) die Ölphase O in einer Menge von ≤ 35 Gew.-%, bezogen auf Gesamtemulsion, enthalten ist.

2. Emulsion nach Anspruch 1, dadurch gekennzeichnet, daß die innere Wasserphase W¹ emulsionsstabilisierende Hilfsmittel, insbesondere NaCl, in einer Konzentration von 0,2 bis 3,0 Gew.-% enthält.

3. Emulsion nach Anspruch 1, dadurch gekennzeichnet, daß die äußere Wasserphase W² hydrophile Emulgatoren eines HLB-Wertes ≥ 13 in einer Konzentration ≥ 0,05 Gew.-% sowie emulsionsstabilisierende Hilfsmittel in einer Konzentration ≥ 0,05 Gew.-% enthält.

4. Emulsion nach Anspruch 1, dadurch gekennzeichnet, daß die äußere Wasserphase W² als hydrophile Emulgatoren solche aus der Gruppe der Amphotenside, Sulfosuccinate, Fettsäuresarcoside, Fettsäure-Eiweiß-Kondensate, Isothionate, Taurate und der aus Zuckern abgeleiteten Tenside enthält.

5. Emulsion nach Anspruch 3, dadurch gekennzeichnet, daß die äußere Wasserphase W² als emulsionsstabilisierende Hilfsmittel Organopolymere aus der Gruppe der Cellulosederivate, der Carboxyl-/Carboxylatgruppen enthaltenden Polyacrylate oder Xanthangum enthält.

6. Emulsion nach Anspruch 1, dadurch gekennzeichnet, daß die innere Wasserphase W¹ und/oder die äußere Wasserphase W² Substanzen mit hautpflegender Wirkung und/oder pharmazeutischer Wirksamkeit enthalten/enthält.

7. Emulsion nach Anspruch 6, dadurch gekennzeichnet, daß sie als Substanzen mit hautpflegender Wirkung und/oder pharmazeutischer Wirksamkeit solche aus der Gruppe Harnstoff, Natriumlactat, Natriumpyrrolidoncarboxylat, Dihydroxyaceton und Panthenol enthält.

8. Emulsion nach Anspruch 1, dadurch gekennzeichnet, daß die Ölphase O Öle und/oder Fette mit hautpflegenden Eigenschaften enthält oder aus diesen besteht.

9. Emulsion nach Anspruch 8, dadurch gekennzeichnet, daß die Ölphase O Öle und/oder Fette mit hautpflegenden Eigenschaften aus der Gruppe Paraffinöle, Vaseline, Fettsäureester und Cyclodimethylsiloxane allein oder in Mischung enthält oder aus diesen besteht.

10. Emulsion nach Anspruch 1, dadurch gekennzeichnet, daß die Ölphase O kosmetische oder pharmazeutische Wirkstoffe, wie Tocopherolacetat, enthält.

## Claims

1. Liquid or pasty, storage-stable multiple emulsion of the W¹/O/W² type, wherein W² forms the external phase of the multiple emulsion, a hydrophobic emulsifier is present at the W¹/O interface and a hydrophilic emulsifier is present at the O/W² interface, characterized in that
(1) the hydrophobic emulsifier has an HLB value of ≤ 8 and is
a₁) a polyacrylic acid ester with long-chain hydrocarbon and polyoxyalkylene groups or
a₂) a polyoxyalkylenepolysiloxane with long-chain alkyl radicals, and the emulsion comprises the hydrophobic emulsifier in an amount of 0.3 to 1.5% by weight, based on the multiple emulsion, and
(2) the hydrophilic emulsifier has an HLB value of > 8 and
(3) the emulsion comprises the oily phase O in an amount of ≤ 35% by weight, based on the total emulsion.

2. Emulsion according to Claim 1, characterized in that the internal agueous phase W¹ comprises emulsion-stabilizing auxiliaries, in particular NaCl, in a concentration of 0.2 to 3.0% by weight.

3. Emulsion according to Claim 1, characterized in that the external aqueous phase W² comprises hydrophilic emulsifiers having an HLB value of ≥ 13 in a concentration of ≥ 0.05% by weight and emulsion-stabilizing auxiliaries in a concentration of ≥ 0.05% by weight.

4. Emulsion according to Claim 1, characterized in that the external aqueous phase W² comprises, as hydrophilic emulsifiers, those from the group consisting of amphosurfactants, sulphosuccinates, fatty acid sarcosides, fatty acid-protein condensates, isethionates, taurates and the surfactants derived from sugars.

5. Emulsion according to Claim 3, characterized in that the external aqueous phase W² comprises, as emulsion-stabilizing auxiliaries, organic polymers from the group consisting of cellulose derivatives, polyacrylates containing carboxyl/carboxylate groups and xanthan gum.

6. Emulsion according to Claim 1, characterized in that the internal aqueous phase W¹ and/or the external aqueous phase W² comprise/comprises substances which have a skin care action and/or pharmaceutical activity.

7. Emulsion according to Claim 6, characterized in that it comprises, as substances having a skin care action and/or pharmaceutical activity, those from the group consisting of urea, sodium lactate, sodium pyrrolidonecarboxylate, dihydroxyacetone and panthenol.

8. Emulsion according to Claim 1, characterized in that the oily phase O comprises oils and/or fats having skin care properties or consists of these.

9. Emulsion according to Claim 8, characterized in that the oily phase O comprises oils and/or fats having skin care properties from the group consisting of paraffin oils, vaseline, fatty acid esters and cyclodimethylsiloxanes, by themselves or as a mixture, or consists of these.

10. Emulsion according to Claim 1, characterized in that the oily phase O comprises cosmetic or pharmaceutical active compounds, such as tocopherol acetate.

## Revendications

1. Emulsion multiple du type E¹/H/E², liquide ou pâteuse, stable pendant le stockage, dans laquelle E² forme la phase externe de l'émulsion multiple, un émulsifiant hydrophobe est présent à l'interface E¹/H et un émulsifiant hydrophile est présent à l'interface H/E², caractérisée en ce que :
(1) l'émulsifiant hydrophobe présente une valeur de HLB (équilibre hydrophile/lipophile) ≤ 8 et est
a₁) un ester de l'acide polyacrylique avec des groupes d'hydrocarbure et des groupes de polyoxyalkylène à chaîne longue, ou
a₂) un polyoxyalkylènepolysiloxane avec des restes alkyles à chaîne longue, et l'émulsifiant hydrophobe est présent en une quantité de 0,3 à 1,5 % en poids par rapport à l'émulsion multiple, et
(2) l'émulsifiant hydrophile présente une valeur de HLB > 8 et
(3) la phase huileuse H est contenue en une quantité ≤ 35 % en poids par rapport à l'émulsion totale.

2. Emulsion selon la revendication 1, caractérisée en ce que la phase aqueuse interne E¹ contient un adjuvant stabilisant l'émulsion, en particulier NaCl, à une concentration de 0,2 à 3,0 % en poids.

3. Emulsion selon la revendication 1, caractérisée en ce que la phase aqueuse externe E² contient des émulsifiants hydrophiles ayant une valeur de HLB ≥ 13, à une concentration ≥ 0,05 % en poids, ainsi que des adjuvants stabilisant l'émulsion, à une concentration ≥ 0,05 % en poids.

4. Emulsion selon la revendication 1, caractérisée en ce que la phase aqueuse externe E² contient, comme émulsifiants hydrophiles, des émulsifiants choisis dans l'ensemble constitué par les agents tensio-actifs amphotères, les sulfosuccinates, les sarcosides d'acide gras, les condensats d'acide gras et de protides, les isothionates, les taurates et les agents tensio-actifs dérivés des sucres.

5. Emulsion selon la revendication 3, caractériseé en ce que la phase aqueuse externe E² contient, comme adjuvants stabilisant l'émulsion, des organopolymères choisis dans l'ensemble constitué par les dérivés cellulosiques, les polyacrylates contenant des groupes carboxyles/carboxylates ou la gomme de xanthane.

6. Emulsion selon la revendication 1, caractérisée en ce que la phase aqueuse interne E¹ et/ou la phase aqueuse externe E² contient/contiennent des substances ayant un effet de soin cutané et/ou un effet pharmaceutique.

7. Emulsion selon la revendication 6, caractérisée en ce qu'elle contient, comme substances ayant un effet de soin cutané et/ou un effet pharmaceutique, celles de l'ensemble constitué par l'urée, le lactate de sodium, le carboxylate sodique de pyrrolidone, la dihydroxyacétone et le panthénol.

8. Emulsion selon la revendication 1, caractérisée en ce que la phase huileuse H contient des huiles et/ou des graisses ayant des propriétés de soin cutané ou est formée de celles-ci.

9. Emulsion selon la revendication 8, caractérisée en ce que la phase huileuse H contient des huiles et/ou des graisses ayant des propriétés de soin cutané choisies dans l'ensemble constitué par les huiles de paraffine, la vaseline, les esters d'acide gras et les cyclodiméthylsiloxanes, individuellement ou mélangées, ou est formée de celles-ci.

10. Emulsion selon la revendication 1, caractérisée en ce que la phase huileuse H contient des principes actifs cosmétiques ou pharmaceutiques, comme l'acétate de tocophérol.
